# FASCICULE DE BREVET EUROPEEN

(11) **EP 0 782 462 B1**
(45) Date de publication et mention de la délivrance du brevet: **09.06.1999**
(21) Numéro de dépôt: 95930565.7
(22) Date de dépôt: 11.09.1995
(51) Int. Cl.: A61M 16/00

(54) **DISPOSITIF D'AIDE RESPIRATOIRE COMMANDE EN PRESSION**
DRUCKGESTEUERTE ATEMHILFSVORRICHTUNG
PRESSURE-CONTROLLED BREATHING AID

(30) Priorité: 12.09.1994 FR 9410839
(43) Date de publication de la demande: 09.07.1997
(73) Titulaire: Nellcor Puritan Bennett France Développement, 91975 Courtabouef Cedex (FR)
(72) Inventeur: BOURDON, Guy, F-91370 Verrières-le-Buisson (FR)
(74) Mandataire: Pontet, Bernard (FR)
(86) Numéro de dépôt international: FR9501158
(87) Numéro de publication internationale: WO9608285

(56) Documents cités:
- WO-A-89/10768
- WO-A-92/11054
- WO-A-93/21982
- WO-A-93/25260
- DE-A- 3 732 475
- US-A- 3 741 208

## Description

La présente invention concerne un dispositif d'aide respiratoire commandé en pression. Un dispositif selon le préambule de la revendication 1 est connu par le document FR-A-2695830.

Les dispositifs d'aide respiratoire - ou dispositifs de ventilation - utilisés couramment dans la ventilation mécanique peuvent se classer en deux groupes principaux, à savoir les appareils volumétriques caractérisés par la délivrance d'un volume déterminé à chaque cycle respiratoire, et les appareils commandés en pression, caractérisés par la délivrance d'une pression déterminée à chaque cycle respiratoire.

Les appareils volumétriques ont l'avantage de garantir un débit respiratoire déterminé, mais ils présentent des inconvénients majeurs. En particulier, ils exposent le patient à des risques de barotraumatisme car ils tendent à appliquer une pression qui croît en fin d'insufflation. En outre, le patient risque de se désadapter à l'appareil en ce sens que les réflexes respiratoires du patient peuvent apparaître à des moments différents de ceux où les volumes imposes par l'appareil finissent d'être délivrés.

Au contraire, les appareils commandés en pression permettent une bien meilleure synchronisation du patient avec l'appareil et d'éviter les risques de barotraumatisme puisque la pression maximum délivrée est connue à l'avance. Par contre, le volume délivré au patient à chaque cycle et le débit respiratoire ne sont pas garantis.

Le but de la présente invention est de proposer un dispositif d'aide respiratoire qui combine les avantages des deux modes de ventilation connus discutés plus haut.

Suivant l'invention, le dispositif d'aide respiratoire en mode pression connu, est caractérisé par les caractéristiques de la seconde partie de la revendication 1.

Ainsi, on règle la pression dans un sens tendant à assurer le volume prédéterminé appliqué comme consigne. De cette façon, on garantit un volume sans prendre le risque de faire augmenter la pression d'une manière incontrôlée, ni créer de risque particulier de désadaptation entre la cadence respiratoire du patient et celle de l'appareil. En particulier, l'invention est parfaitement compatible avec les dispositifs du genre décrits dans le FR-A-2 695 830 dans lesquels l'appareil détecte les réflexes respiratoires du patient pour passer des phases inspiratoires aux phases expiratoires et inversement. Le dispositif connu par EP-A-419551 différe de ce dispositif en ce que la pression régnant dans la branche n'est par réglée.

Pour éviter tout risque de barotraumatisme, il est avantageux de prévoir des moyens pour fixer une pression maximum prédéterminée que la pression appliquée au patient ne pourra pas dépasser même si le volume ou le débit délivré est insuffisant.

Il est également avantageux de prévoir un dispositif de signalisation ou autre alarme détectant l'occurrence simultanée de l'insuffisance du débit et du réglage de la pression à sa valeur maximum prédéterminée, pour signaliser cette situation d'incapacité de l'appareil à assurer le débit respiratoire fixé comme consigne.

Dans le cadre de la présente invention, on emploie l'expression "débit respiratoire" pour désigner aussi bien le débit de gaz respirable inspiré ou expiré par unité de temps, que le volume ou la quantité de gaz inspiré ou expiré par cycle respiratoire.

De préférence, les moyens de régulation appliquent à la pression inspiratoire une variation de pression qui est égale en pourcentage à l'écart entre le débit inspiratoire et la consigne.

Toutefois, dans le cas où un extremum de pression est prévu et si l'application d'une telle variation conduirait à franchir l'extremum, la nouvelle pression inspiratoire est rendue égale à l'extremum de pression.

D'autres particularités et avantages de l'invention ressortiront encore de la description ci-après, relative à des exemples non limitatifs.

Aux dessins annexés :
- la figure 1 est un schéma d'un premier mode de réalisation du dispositif selon l'invention ;
- la figure 2 est un organigramme de fonctionnement des moyens de régulation du dispositif de la figure 1 ; et
- les figures 3 et 4 sont deux schémas analogues à la figure 1 mais relatifs à deux modes de réalisation du dispositif selon l'invention.

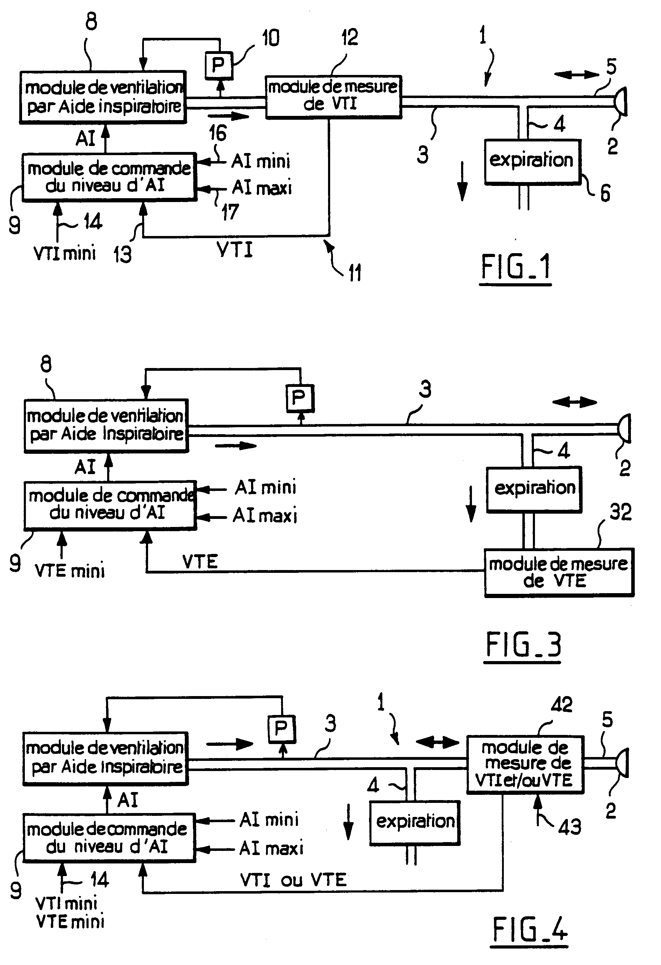

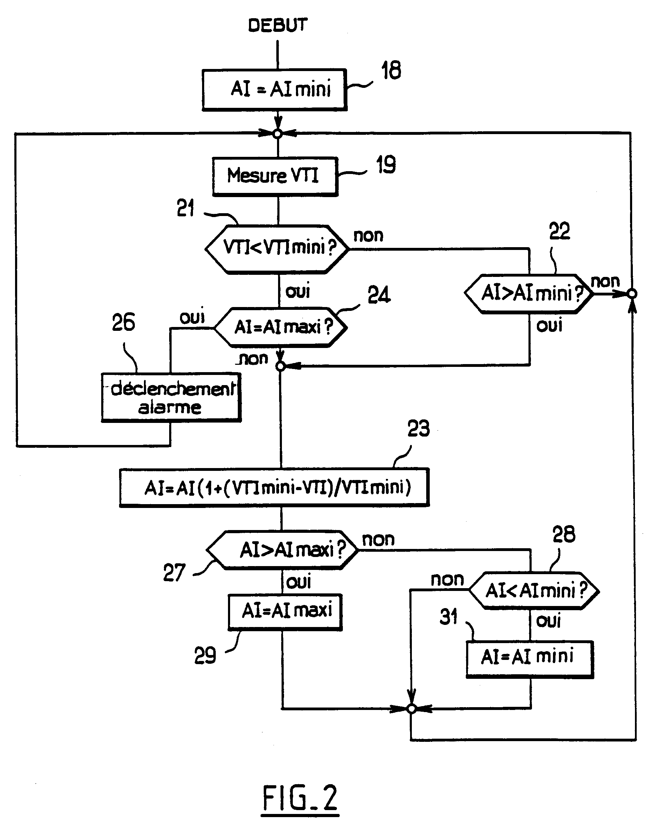

Dans l'exemple représenté à la figure 1, le dispositif d'aide respiratoire comprend un circuit patient 1 comprenant à son tour un raccordement patient 2, à savoir un masque facial, ou nasal, ou une sonde d'intubation ou de trachéotomie, raccordé à une branche inspiratoire 3 et à une branche expiratoire 4 par l'intermédiaire d'une branche bidirectionnelle 5. La branche expiratoire 4 comporte un dispositif d'expiration 6 qui, de manière non représentée, comporte une valve d'expiration et des moyens de commande de cette valve. Pendant les phases inspiratoires de la respiration du patient, la valve d'expiration est fermée. Pendant les phases expiratoires de la respiration du patient, la valve d'expiration peut être soit ouverte pour que le patient expire à la pression atmosphérique, soit fonctionner à la manière d'un clapet de décharge pour obliger le patient à expirer sous une certaine surpression prédéterminée.

La branche inspiratoire 3 est reliée, à son extrémité opposée au masque 2, à un module de ventilation par aide inspiratoire 8 qui comprend des moyens, tels qu'un groupe moto-turbine à vitesse réglable, pour délivrer du gaz respirable à travers la branche inspiratoire 3 sous pression réglable, en direction du masque 2, des moyens pour détecter les réflexes respiratoires du patient, par exemple d'après les variations de débit instantané, et des moyens pour commander la valve d'expiration des moyens d'expiration 6 et une valve d'inspiration placée sur la branche inspiratoire 3 pour ouvrir la valve d'inspiration et fermer la valve d'expiration pendant les phases inspiratoires, et fermer la valve d'inspiration et libérer la valve d'expiration pendant les phases expiratoires. Ainsi, en phase inspiratoire, le patient communique de manière étanche avec la branche inspiratoire 3 et le volume circulant dans la branche inspiratoire 3 correspond au volume de gaz inspiré. Et pendant les phases expiratoires, le patient communique de manière étanche avec la branche expiratoire 4 et le volume circulant dans la branche expiratoire 4 correspond au volume de gaz expiré.

De tels dispositifs d'aide inspiratoire, ou des dispositifs d'aide inspiratoire du même genre, sont décrits dans l'art antérieur en particulier dans le FR-A-2 695 830.

Le module de ventilation 8 peut comporter des moyens d'asservissement en pression par lesquels la pression P détectée dans la branche inspiratoire 3 par un détecteur 10 est comparée à une consigne de pression AI pour régler par exemple la vitesse de rotation du groupe mototurbine dans le sens tendant à rendre la pression P égale à la consigne AI.

Suivant l'invention, le dispositif d'aide respiratoire comprend des moyens de régulation 11 du débit respiratoire du patient. Les moyens de régulation 11 comprennent un module 9 de commande de la consigne de pression AI que le module de ventilation 8 doit appliquer à la branche inspiratoire 3 pendant les phases inspiratoires.

Les moyens de régulation 11 comprennent en outre un module 12 de mesure du volume VTI inspiré par le patient au cours de chaque cycle respiratoire. Le module 12 fournit au module de commande 9 un signal indicatif du volume VTI. Le module de commande 9 comprend une entrée 13 pour recevoir le signal VTI, et trois entrées 14, 16, 17, permettant à l'utilisateur du dispositif d'entrer dans le module de commande une consigne de débit respiratoire minimum, sous la forme d'un minimum de volume inspiré par cycle VTImini, une consigne de pression inspiratoire minimum AImini, et une consigne de pression inspiratoire maximum AImaxi.

D'une manière générale, le module de commande 9 compare le débit mesuré VTI avec la consigne VTImini et ajuste la consigne de pression AI dans le sens tendant à rapprocher le débit mesuré VTI de la consigne VTImini, sans toutefois faire sortir la consigne AI de l'intervalle compris entre les deux extremum AImini et AImaxi. Dans cet intervalle, le module de commande 9 tend à faire augmenter la consigne AI lorsque le volume VTI mesuré est inférieur à la consigne VTImini, et à réduire la consigne de pression AI dans le cas contraire.

A la mise en route de l'appareil, on choisit les consignes VTImini et AImini telles que le débit respiratoire VTI s'établisse à une valeur supérieure à VTImini lorsque la consigne de pression AI est égale AImini. Ainsi, si le patient respire comme prévu, la consigne de pression AI se stabilise à AImini avec un débit respiratoire supérieur à la consigne de minimum VTImini. Ce n'est qu'en cas d'anomalie ou incident respiratoire, par exemple obstruction partielle des voies respiratoires, que le débit respiratoire mesuré VTI est susceptible de devenir inférieur à VTImini, provoquant ainsi une augmentation de la consigne AI établie par le module de commande 9. Lorsque la respiration redevient normale, le débit respiratoire redevient supérieur à la consigne VTImini, de sorte que le module de commande 9 ramène plus ou moins rapidement la consigne de pression AI à la valeur AImini.

On va maintenant décrire plus en détail, en référence à la figure 2, l'organigramme mis en oeuvre par le module de commande 9. Au début, AI est rendu égal à AImini (étape 18).

Ensuite, à la fin de chaque cycle respiratoire, ou au cours de chaque phase expiratoire, la mesure VTI du volume inspiré lors de la phase inspiratoire précédente est acquise (étape 19) puis comparée à la consigne VTImini par le test 21. Si le volume mesuré VTI est supérieur ou égal à VTImini, autrement dit si le volume inspiré par le patient est satisfaisant, un test 22 détermine si la consigne de pression AI est ou non supérieure au minimum AImini. Si la consigne de pression est égale au minimum, on est donc dans les conditions idéales (volume au moins égal au minimum, pression minimum), et on retourne donc directement à l'étape 19 d'acquisition de la mesure du volume inspiré suivant. Dans le cas contraire, on va profiter du fait que le volume inspiré est satisfaisant pour tenter de réduire la consigne de pression par une étape 23 dans laquelle on applique à la consigne de pression AI, exprimée en valeur relative, une variation égale en pourcentage et opposée en signe à l'écart entre le volume inspiré mesuré VTI et la consigne VTImini. La formule est telle que dans le cas particulier où le volume mesuré VTI est égal à VTImini, aucune modification n'est appliquée à la consigne de pression AI (variation de 0 %).

En revenant maintenant au test 21 sur le volume mesuré VTI, si celui-ci est inférieur à la consigne VTImini, on va chercher à augmenter la consigne de pression AI pour assister davantage le patient. Mais au préalable, par un test 24, on va vérifier si la consigne de pression AI n'atteint pas déjà le maximum AImaxi. Si oui, une alarme est déclenchée (étape 26) pour signaler la nécessité d'une intervention urgente.

Au contraire, si la consigne de pression AI n'est pas encore égale à AImaxi, on se rend comme précédemment à l'étape 23 par laquelle il va être appliqué à la consigne AI une variation égale en pourcentage et opposée en signe à l'écart entre le volume VTI mesuré et la consigne VTImini.

Avant d'appliquer effectivement à l'entrée du module de ventilation 8, la consigne AI diminuée ou augmentée telle qu'elle a été calculée à l'étape 23, on va d'abord vérifier par un test 27 si la nouvelle valeur AI calculée ne dépasse pas le maximum AImaxi, et par un test 28 qu'elle n'est pas inférieure au minimum AImini.

Si la nouvelle valeur AI franchit l'un ou l'autre de ces extremum, la consigne AI qui sera appliquée au module de ventilation 8 sera égale à l'extremum en question (étapes 29 et 31).

L'exemple de la figure 3 ne sera décrit que pour ses différences par rapport à celui de la figure 1.

Dans l'exemple de la figure 3, le débit respiratoire n'est plus mesuré à travers le volume inspiré à chaque cycle, mais à travers le volume VTE expiré à chaque cycle. Pour cela, on a supprimé le module de mesure de VTI 12 sur la branche inspiratoire 3, et on l'a remplacé par un module de mesure de VTE 32 sur la branche expiratoire 4.

La consigne de débit respiratoire minimum appliquée au module de commande 9 est alors une consigne VTEmini de volume expiré par cycle, de manière à pouvoir être comparée directement à la mesure fournie par le module 32.

Il peut y avoir intérêt à sélectionner cas par cas la mesure sur le volume inspiré ou la mesure sur le volume expiré. C'est la solution proposée par le mode de réalisation de la figure 4, qui ne sera décrit que pour ses différences par rapport à celui de la figure 1.

Le module de mesure 42 est cette fois installé dans la branche bidirectionnelle 5 du circuit patient 1 et il comporte des moyens 43 pour sélectionner le sens de l'écoulement dont le volume est à mesurer. En fonction de cette sélection, le module 42 fournit au choix une mesure de VTI ou VTE. En fonction du mode de fonctionnement du module de mesure 42, le module de commande 9 interprète comme une consigne de volume inspiré ou comme une consigne de volume expiré l'entrée appliquée en 14. Il n'y a plus de module de mesure sur la branche inspiratoire 3 ni sur la branche expiratoire 4.

Dans tous les modes de réalisation décrits, la rapidité d'exécution de l'organigramme de la figure 2 est suffisante pour que la mesure de volume effectuée à chaque cycle respiratoire permette de corriger la pression appliquée lors de la phase inspiratoire suivante. Lorsque la mesure porte sur le volume expiré, il est cependant possible que la correction de pression n'intervienne qu'en cours, et non dès le début, de la phase inspiratoire suivante.

L'invention est applicable à tous ventilateurs capables de mesurer les volumes ou débits délivrés et de piloter automatiquement la valeur de la pression d'insufflation.

L'invention est applicable à tous modes de ventilation par commande de la pression, et notamment aux modes "aide inspiratoire" et "pression contrôlée". L'aide inspiratoire est un mode consistant à maintenir pendant l'insufflation une pression sensiblement constante dans le circuit patient, le patient déclenchant le début et la fin de l'insufflation par ses réflexes respiratoires. Le mode pression contrôlée est identique au mode aide inspiratoire, excepté que le patient ne déclenche pas la fin de l'insufflation, celle-ci étant déterminée par un temps fixe.

## Revendications

1. Dispositif d'aide respiratoire commandé en pression, comprenant des moyens détectant pendant le fonctionnement du dispositif l'activité respiratoire d'un patient et pour générer, en fonction de cette activité, des phases d'inspiration et d'expiration synchronisées avec ladite activité, des moyens (8) alimentant en gaz respirable, pendant les phases d'inspiration pendant le fonctionnement du dispositif, une branche inspiratoire (3) d'un circuit patient (1) sous une pression inspiratoire régulée relativement à une consigne (AI) pour la pression inspiratoire régnant dans la branche inspiratoire (3) caractérisé par :
- des moyens (12 ; 32 ; 42) pour mesurer le débit respiratoire (VTI ; VTE),
- des moyens pour comparer le débit respiratoire (VTI ; VTE) à une consigne de débit (VTImini ; VTEmini); et
- des moyens de régulation (11) pour augmenter la consigne de pression inspiratoire (AI) dans des cas de débit respiratoire inférieur à la consigne de débit (VTImini ; VTEmini) et diminuer la consigne de pression inspiratoire dans des cas de débit respiratoire supérieur à la consigne de débit (VTImini ; VTEmini), de sorte que ladite consigne (AI) est ajustée pour respecter une condition de débit.

2. Dispositif selon la revendication 1, caractérisé en ce que les moyens (12 ; 32 ; 42) pour mesurer le débit respiratoire mesurent le volume respiré (VTI ; VTE) par le patient lors d'un cycle respiratoire, et les moyens de régulation (11) se basent sur le résultat de la comparaison (21) de ce volume avec la consigne de débit pour régler la pression inspiratoire appliquée lors d'un cycle suivant.

3. Dispositif selon la revendication 1 ou 2, caractérisé en ce que les moyens (12 ; 42) pour mesurer le débit respiratoire mesurent le débit inspiré (VTI) par le patient.

4. Dispositif selon la revendication 1 ou 2, caractérisé en ce que les moyens (32 ; 42) pour mesurer le débit respiratoire mesurent le débit expiré (VTE) par le patient.

5. Dispositif selon la revendication 1 ou 2, caractérisé en ce que les moyens (42) pour mesurer le débit respiratoire mesurent sélectivement le débit inspiré (VTI) ou le débit expiré (VTE) par le patient.

6. Dispositif selon la revendication 3, caractérisé en ce qu'il comprend des moyens (8) pour mettre la branche inspiratoire (3) en communication sensiblement étanche avec les voies respiratoires du patient pendant des phases inspiratoires du cycle respiratoire et pour interrompre la circulation de gaz respirable dans la branche inspiratoire (3) pendant des phases expiratoires du cycle respiratoire, et en ce que les moyens (12) pour mesurer le débit respiratoire sont reliés à la branche inspiratoire (3).

7. Dispositif selon la revendication 4, caractérisé en ce que le circuit patient (1) comprend une branche expiratoire (4), et en ce que le dispositif comprend des moyens (5) pour mettre la branche expiratoire (4) en communication sensiblement étanche avec les voies respiratoires du patient pendant des phases expiratoires du cycle respiratoire et pour interrompre la circulation de gaz dans la branche expiratoire (4) pendant des phases inspiratoires du cycle respiratoire, et en ce que les moyens (32) pour mesurer le débit respiratoire sont reliés à la branche expiratoire (4).

8. Dispositif selon la revendication 5, caractérisé en ce que le circuit patient (1) comprend une branche bidirectionnelle (5) à laquelle sont raccordés la branche inspiratoire (3) et un trajet d'expiration (4), en ce que les moyens (42) pour mesurer le débit respiratoire sont reliés à la branche bidirectionnelle et sont capables de mesurer le débit dans les deux sens d'écoulement, des moyens (43) étant prévus pour sélectionner le sens de l'écoulement dont les moyens de mesure du débit respiratoire (42) mesurent le débit.

9. Dispositif selon l'une des revendications 1 à 8, caractérisé en ce que les moyens de régulation augmentent la pression inspiratoire (AI) dans lesdits cas de débit respiratoire (VTI, VTE) inférieur à la consigne de débit si la consigne de pression inspiratoire (AI) est inférieure à une pression maximum prédéterminée (Aimaxi).

10. Dispositif selon l'une des revendications 1 à 9, caractérisé en ce que les moyens de régulation diminuent la pression inspiratoire (AI) dans lesdits cas de débit respiratoire (VTI, VTE) supérieur à la consigne de débit si la consigne de pression inspiratoire (AI) est supérieure à une pression minimum prédéterminée (Aimini).

11. Dispositif selon l'une des revendications 1 à 10, caractérisé en ce que dans certains au moins desdits cas de débit respiratoire (VTI ; VTE) inférieur ou supérieur à la consigne de débit (VTImini ; VTEmini), les moyens de régulation appliquent à la pression inspiratoire une variation de pression qui augmente avec l'écart entre le débit respiratoire mesuré (VTI ; VTE) et la consigne de débit(VTImini ; VTEmini).

12. Dispositif selon la revendication 11, caractérisé en ce que ladite variation de pression est égale en pourcentage à l'écart entre le débit respiratoire mesuré (VTI ; VTE) et la consigne de débit (VTImini ; VTEmini).

13. Dispositif selon l'une des revendications 1 à 10, caractérisé en ce que dans certains au moins desdits cas de débit respiratoire (VTI ; VTE) inférieur ou supérieur à la consigne de débit (VTI mini ; VTE mini), les moyens de régulation calculent une pression modifiée et appliquent la pression modifiée si la pression modifiée reste en-deça d'un extremum prédéterminé et rendent la pression inspiratoire égale à l'extremum prédéterminé si la pression modifiée calculée franchit l'extrum prédeterminé.

14. Dispositif selon la revendication 9 ou 13, caractérisé en ce qu'il comprend un moyen (26) pour signaliser l'occurrence simultanée d'un débit respiratoire (VTI ; VTE) inférieur à la consigne de débit (VTImini) et d'une pression inspiratoire (AI) au moins égale à une pression maximum prédéterminée.

## Claims

1. Pressure mode breathing aid device, comprising means of detecting during operation of the device the breathing activity of a patient and of generating, as a function of that activity, inspiration and expiration phases synchronised with the said activity, means (8) for supplying during operation of the device breathable gas, during the inspiration phases, to an inspiratory branch (3) of a patient circuit (1) at an inspiratory pressure adjusted in relation to a command (AI) for the inspiratory pressure prevailing in the inspiratory branch (3) characterized by:
- means (12; 32; 42) of measuring the breathed volume (VTI, VTE),
- means of comparing the breathed volume (VTI; VTE) with a volume command (VTImini; VTEmini), and
- regulation means (11) to increase the inspiratory pressure command (AI) in the case of a breathed volume lower than the volume command (VTImini; VTEmini), and to reduce the inspiratory pressure command in the case of a breathed volume higher than the volume command (VTImini; VTEmini), so that said command (AI) is adjusted for fulfilling a flow rate condition.

2. Device according to Claim 1, characterized in that the means (12; 32; 42) for measuring the breathed volume measure the volume amount (VTI; VTE) breathed by the patient during a breathing cycle, and the regulating means (11) are based on the result of the comparison (21) of this volume amount with the volume command in order to adjust the inspiratory pressure applied during a following cycle.

3. Device according to Claim 1 or 2, characterized in that the means (12; 42) of measuring the breathed volume measure the volume (VTI) inspired by the patient.

4. Device according to Claim 1 or 2, characterized in that the means (32; 42) of measuring the breathed volume measure the volume (VTE) expired by the patient.

5. Device according to Claim 1 or 2, characterized in that the means (42) of measuring the breathed volume selectively measure the volume inspired (VTI) or the volume expired (VTE) by the patient.

6. Device according to Claim 3, characterized in that it comprises means (8) for connecting the inspiratory branch (3) in substantially gas-tight manner with the respiratory channels of the patient during inspiratory phases of the respiratory cycle and to interrupt the flow of breathable gas in the inspiratory branch (3) during expiratory phases of the respiratory cycle, and in that the means (12) of measuring the breathed volume are connected to the inspiratory branch (3).

7. Device according to Claim 4, characterized in that the patient circuit (1) comprises an expiratory branch (4) and in that the device comprises means (5) of connecting the expiratory branch (4) in a substantially gas-tight manner with the respiratory channels of the patient during expiratory phases of the respiratory cycle and to interrupt the flow of gas in the expiratory branch (4) during inspiratory phases of the respiratory cycle, and in that the means (32) of measuring the breathed volume are connected to expiratory branch (4).

8. Device according to Claim 5, characterized in that the patient circuit (1) comprises a bi-directional branch (5) to which are connected the inspiratory branch (3) and an expiration path (4), in that the means (42) of measuring the breathed volume are connected to the bi-directional branch and are capable of measuring the volume in both directions of flow, means (43) being provided to select the direction of the flow in which the breathed volume measuring means (42) measure the volume.

9. Device according to one of Claims 1 to 8, characterized in that the regulating means increase the inspiratory pressure (AI) in the said cases of a breathed volume (VTI, VTE) less than the volume command if the inspiratory pressure command (AI) is less than a predetermined maximum pressure (AImaxi).

10. Device according to one of Claims 1 to 9, characterized in that the regulating means reduce the inspiratory pressure (AI) in the said cases of a breathed volume (VTI, VTE) higher than the volume command if the inspiratory pressure (AI) is greater than a predetermined minimum pressure (AImini).

11. Device according to one of Claims 1 to 10, characterized in that, in at least certain of the said breathed volume (VTI; VTE) cases below and above the volume command (VTImini; VTEmini), the regulating means apply to the inspiratory pressure a pressure variation which increases with the difference between the measured breathed volume (VTI, VTE) and the volume command (VTImini, VTEmini).

12. Device according to Claim 11, characterized in that the said pressure variation is equal in percentage to the difference between the measured breathed volume (VTI; VTE) and the volume command (VTImini; VTEmini).

13. Device according to one of Claims 1 to 10, characterized in that, in at least certain of the said cases of breathed volume (VTI; VTE) below or above the volume command (VTImini; VTEmini), the regulating means compute a modified pressure and apply the modified pressure if the modified pressure does not go beyond a predetermined extreme value and make the inspiratory pressure equal to the predetermined extreme value if the computed modified pressure goes beyond the predetermined extreme value.

14. Device according to Claim 9 or 13, characterized in that it comprises a means (26) of indicating the simultaneous occurrence of a breathed volume (VTI; VTE) below the volume command (VTImini) and an inspiratory pressure (AI) at least equal to a predetermined maximum pressure.

## Patentansprüche

1. Druckgeregeltes Atemhilfsgerät, das folgendes enthält: Einrichtungen, die während des Betriebs des Gerätes die Atmungsaktivität eines Patienten überwachen, um in Abhängigkeit von dieser Tätigkeit mit der Tätigkeit synchron verlaufende Einatmungs- und Ausatmungsphasen zu erzeugen, Einrichtungen (8), die während des Betriebs des Gerätes während der Einatmungsphasen Atemgas zuführen, einen Einatmungszweig (3) eines Patienten-Leitungskreises (1) unter einem Einatmungsdruck, der relativ zu einem eingestellten Wert [AI] für den im Einatmungszweig (3) herrschenden Einatmungsdruck geregelt wird, gekennzeichnet durch:
- Einrichtungen (12; 32; 42) zum Messen der Atemleistung (VTI; VTE),
- Einrichtungen zum Vergleich der Atemleistung (VTI; VTE) mit einem eingestellten Leistungswert (VTImini; VTEmini); und
- Regeleinrichtungen (11) um den eingestellten Wert für den Einatmungsdruck (AI) zu erhöhen, wenn die Atemleistung unter dem eingestellten Leistungswert (VTImini; VTEmini) liegt, und um den eingestellten Wert für den Einatmungsdruck (AI) zu vermindern, wenn die Atemleistung über dem eingestellten Leistungswert (VTImini; VTEmini) liegt, so daß der eingestellte Wert [AI] zum Einhalten eines Leistungszustandes eingestellt wird.

2. Gerät nach Anspruch 1, dadurch gekennzeichnet, daß die Einrichtungen (12; 32; 42) zum Messen der Atemleistung das Atemvolumen (VTI; VTE) eines Patienten während eines Atemcyclus messen und die Regeleinrichtungen (11) sich auf das Ergebnis des Vergleichs (21) dieses Volumens mit dem eingestellten Leistungswert beziehen, um den während eines Folgendencyclus angelegten Einatmungsdruck zu regeln.

3. Gerät nach Anspruch 1 oder 2, dadurch gekennzeichnet, daß die Einrichtungen (12; 42) zum Messen der Atemleistung die vom Patienten eingeatmete Menge (VTI) messen.

4. Gerät nach Anspruch 1 oder 2, dadurch gekennzeichnet, daß die Einrichtungen (32; 42) zum Messen der Atemleistung die vom Patienten ausgeatmete Menge (VTE) messen.

5. Gerät nach Anspruch 1 oder 2, dadurch gekennzeichnet, daß die Einrichtungen (42) zum Messen der Atemleistung selektiv die vom Patienten eingeatmete (VTI) oder ausgeatmete Menge (VTE) messen.

6. Gerät nach Anspruch 3, dadurch gekennzeichnet, daß es Einrichtungen (8) enthält, um den Einatmungszweig (3) während Einatmungsphasen des Atemcyclus in eine im Wesentlichen dichte Verbindung mit den Atemwegen des Patienten zu bringen und um die Zirkulation des Atmungsgases im Einatmungszweig (3) während der Ausatmungsphasen des Atemcyclus zu unterbrechen, und dadurch daß die Einrichtungen (12) zum Messen der Atemleistung mit dem Einatmungszweig (3) verbunden sind.

7. Gerät nach Anspruch 4, dadurch gekennzeichnet, daß der Patienten-Leitungskreis (1) einen Ausatmungszweig (4) enthält und dadurch daß das Gerät Mittel (5) enthält, um den Ausatmungszweig (4) während der Ausatmungsphasen des Atemcyclus in eine im Wesentlichen dichte Verbindung mit den Atemwegen des Patienten zu bringen und um die Gaszirkulation im Ausatmungszweig (4) während der Einatmungsphasen des Atemcyclus zu unterbrechen, und dadurch daß die Einrichtungen (32) zum Messen der Atemleistung mit dem Ausatmungszweig (4) verbunden sind.

8. Gerät nach Anspruch 5, dadurch gekennzeichnet, daß der Patienten-Leitungskreis (1) einen Zweirichtungszweig (5), an den der Einatmungszweig (3) und eine Ausatmungsleitung (4) angeschlossen sind, enthält, dadurch daß die Einrichtungen (42) zum Messen der Atemleistung mit dem Zweirichtungszweig verbunden sind und die Leistung in beiden Strömungsrichtungen zu messen vermögen, wobei Einrichtungen (43) zum Wählen der Strömungsrichtung, deren Leistung die Meßeinrichtungen für die Atemleistung (42) messen, vorgesehen sind.

9. Gerät nach einem der Ansprüche 1 bis 8, dadurch gekennzeichnet, daß die Regeleinrichtungen den Einatmungsdruck (AI) in den Fällen erhöhen, in denen die Atemleistung (VTI, VTE) unter dem eingestellten Leistungswert liegt, wenn der eingestellte Wert des Einatmungsdruckes (AI) unter einem zuvor festgelegten Maximaldruck (Aimaxi) liegt.

10. Gerät nach einem der Ansprüche 1 bis 9, dadurch gekennzeichnet, daß die Regeleinrichtungen den Einatmungsdruck (AI) in den Fällen vermindern, in denen die Atemleistung (VTI, VTE) über dem eingestellten Leistungswert liegt, wenn der eingestellte Wert des Einatmungsdruckes (AI) über einem zuvor festgelegten Minimaldruck (Aimini) liegt.

11. Gerät nach einem der Ansprüche 1 bis 10, dadurch gekennzeichnet, daß in wenigstens einigen der Fälle, in denen die Atemleistung (VTI; VTE) unter oder über dem eingestellten Leistungswert (VTImini; VTImaxi) liegt, die Regeleinrichtungen am Einatmungsdruck eine Druckveränderung vornehmen, die sich mit der Differenz zwischen der gemessenen Atemleistung (VTI; VTE) und dem eingestellten Leistungswert (VTImini; VTEmini) vergrößert.

12. Gerät nach Anspruch 11, dadurch gekennzeichnet, daß die Druckveränderung in Prozent der Differenz zwischen der gemessenen Atemleistung (VTI; VTE) und dem eingestellten Leistungswert (VTImini; VTEmini) entspricht.

13. Gerät nach einem der Ansprüche 1 bis 10, dadurch gekennzeichnet, daß in wenigstens einigen Fällen, in denen die Atemleistung (VTI; VTE) unter oder über dem eingestellten Leistungswert (VTImini; VTImaxi) liegt, die Regeleinrichtungen einen veränderten Druck berechnen und den veränderten Druck anlegen, wenn der veränderte Druck nicht an einen zuvor festgelegten Extremwert heranreicht, und den Einatmungsdruck dem zuvor festgelegten Extremwert angleichen, wenn der veränderte berechnete Druck den zuvor festgelegten Extremwert durchbricht.

14. Gerät nach Anspruch 9 oder 13, dadurch gekennzeichnet, daß es eine Einrichtung (26) enthält, um das gleichzeitige Auftreten einer Atemleistung (VTI; VTE), die unter dem eingestellten Leistungswert liegt, und eines Einatmungsdruckes (AI), der wenigstens einem zuvor festgelegten Maximaldruck entspricht, zu melden.
